# EUROPEAN PATENT APPLICATION

(11) **EP 0 965 341 A2**
(43) Date of publication of application: **22.12.1999**
(21) Application number: 99303489.1
(22) Date of filing: 04.05.1999
(51) Int. Cl.: A61K 31/44

(54) **Combination of dofetilide and a calcium channel blocker**

(30) Priority: 14.05.1998 US 85496 P
(71) Applicant: Pfizer Products Inc., Groton, Connecticut 06340 (US); BOSTON UNIVERSITY, Boston, MA 02215 (US)
(72) Inventor: Friedrich, Tilman, Groton Long Point 06340, Connecticut (US); Billing, Clare B., Jr., Mystic 06355, Connecticut (US); Falk, Rodney H., Newton 02159, Massachusetts (US)
(74) Representative: Baillie, Iain Cameron

(57) **Abstract**

Pharmaceutical compositions and methods comprising dofctilide and calcium channel blockers. The compositions and methods are useful for the treatment of arrhythmias.

## Description

### BACKGROUND OF INVENTION

This invention relates to pharmaceutical combination of dofetilide and a calcium channel blocker, kits containing such combinations and the use of such combinations to treat atrial fibrillation in mammals.

Dofetilide is a selective inhibitor of the rapid component of the delayed rectifier potassium current which prolongs the action potential duration and the effective refractory period in a concentration dependent manner. Clinical studies have demonstrated that dofetilide is effective in treating patients with atrial as well as ventricular arrhythmias. Specifically, for example, dofetilide has been studied in patients with severe left ventricular dysfunction (William M. Bailey et al., Electrophysiologic and Hemodynamic Effects of Dofetilide in Patients with Severe Left Ventricular Dysfunction, Circulation, 1992, Vol. 86, Part 4, Supplement 1, page 1265) in which it was found that, unlike class I agents, dofetilide is safe in patients with congestive heart failure.

Studies have shown that episodes of atrial fibrillation result in acute shortening of the atrial refractory period. This is known as "electrical remodeling" of the atrium and is reversible after conversion to sinus rhythm. Prior to complete reversal of the decreased refractory period, the atrium has increased vulnerability for relapse to atrial fibrillation. However, Tieleman et al., in A Clinical Illustration of Reduction of Electrical Remodeling by the use of Intracellular Calcium Lowering Drugs During AF, NASPE Abstract, Pace, Vol. 20, page 1142, No. 368, April 1997 concluded "[p]atients using intracellular calcium lowering drugs during AF seem to experience a lower incidence of relapse of AF after cardioversion. This could be explained by a reduction of electrical remodeling of the atria by prevention of intracellular calcium overload during AF." In addition, Daoud et al., in Circulation, vol. 96, No.5, Sept. 1997 "Effect of Verapamil and Procainamide on Atrial Fibrillation-Induced Electrical Remodeling in Humans" concluded "pretreatment with the calcium channel antagonist verapamil, but not the sodium channel antagonist procainamide, markedly attenuates acute, AF-induced changes in atrial electrophysiological properties. These data suggest that calcium loading during AF may be at least partially responsible for AF-induced electrical remodeling."

Amlodipine is a calcium channel blocker that has seen widespread acceptance. It is approved for the treatment of hypertension. Further, U.S. Pat. No. 5,155,120 discloses its use for the treatment of congestive heart failure.

Although there are some therapies for the treatment of atrial fibrillation there is a continuing search in this field of art for new therapies.

### SUMMARY OF THE INVENTION

This invention is directed to pharmaceutical compositions comprising dofetilide or a pharmaceutically acceptable salt thereof and a calcium channel blocker and for the use of such compositions for the treatment of arrhythmia, including atrial fibrillation, in mammals (e.g., humans either male or female).

The combinations comprise therapeutically effective amounts of dofetilide and a calcium channel blocker.

Another aspect of this invention is a method for treating arrhythmias in a mammal comprising administering to a mammal in need of treatment thereof therapeutically effective amounts of
a. dofetilide; and
b. a calcium channel blocker.

A preferred method is wherein atrial fibrillation is treated.

It is especially preferred that maintenance of normal sinus rhythm is improved. A particularly preferred method is the inhibition of electrical remodeling of the atrium prior to conversion to sinus rhythm.

A particularly preferred mammal is a female or male human.

It is particularly preferred that the human patient has sustained atrial fibrillation.

Another preferred aspect of this method is wherein the calcium channel blocker is administered prior to administering dofetilide.

In another aspect of this method the above sequential administration is followed by substantially simultaneous administration of the calcium channel blocker and dofetilide.

Another aspect of this invention is a kit comprising:
a. a therapeutically effective amount of dofetilide and a pharmaceutically acceptable carrier in a first unit dosage form;
b. a therapeutically effective amount of a calcium channel blocker and a pharmaceutically acceptable carrier in a second unit dosage form; and
c. means for containing said first and second dosage forms.

Another aspect of this invention is a synergistic pharmaceutical composition for improving the maintenance of normal sinus rhythm in a mammal comprising
a. dofetilide; and
b. a calcium channel blocker,
wherein the amount of dofetilide alone and the amount of the calcium channel blocker alone is insufficient to achieve the improved maintenance of normal sinus rhythm if administered alone and wherein the combined effect of the amount of dofetilide and the calcium channel blocker is greater than the sum of the sinus rhythm maintenance effects achievable with individual amounts of the dofetilide and calcium channel blocker; and
c. a pharmaceutically acceptable diluent or carrier.

Yet another aspect of this invention is a synergistic method for improving the maintenance of normal sinus rhythm in a mammal comprising administering to such a mammal
a. an amount dofetilide; and
b. an amount of a calcium channel blocker
wherein the amount of dofetilide alone and the amount of the calcium channel blocker alone is insufficient to achieve the improved maintenance of normal sinus rhythm if administered alone and wherein the combined effect of the amount of dofetilide and the calcium channel blocker is greater than the sum of the sinus rhythm maintenance effects achievable with individual amounts of the dofetilide and calcium channel blocker.

In each of the above compositions, methods and kits, a preferred amount of dofetilide is about 0.1 mcg/kg/day to about 30 mcg/kg/day and an especially preferred amount of dofetilide is about 1 mcg/kg/day to about 15 mcg/kg/day.

In each of the above compositions, methods and kits, a preferred amount of calcium channel blocker is about 0.001 mg/kg/day to about 10 mg/kg/day and an especially preferred amount of calcium channel blocker is about 0.01 mg/kg/day to about 5 mg/kg/day.

In each of the above compositions, methods and kits, especially preferred calcium channel blockers are amlodipine, nifedipine, isradipine, diltiazem or verapamil or a pharmaceutically acceptable salt thereof.

In each of the above compositions, methods and kits, especially preferred calcium channel blockers are amlodipine, nifedipine or isradipine or a pharmaceutically acceptable salt thereof.

In each of the above compositions, methods and kits, an especially preferred calcium channel blocker is amlodipine or a pharmaceutically acceptable salt thereof, particularly the besylate salt thereof.

Another aspect of this invention is a method for treating arrhythmias in a mammal comprising administering to a mammal in need of treatment thereof a therapeutically effective amount of amlodipine or a pharmaceutically acceptable salt thereof, particularly the besylate salt thereof.

A preferred method is wherein atrial fibrillation is treated.

It is especially preferred that maintenance of normal sinus rhythm is improved.

A particularly preferred method is the inhibition of electrical remodeling of the atrium prior to conversion to sinus rhythm.

A particularly preferred mammal is a female or male human.

A preferred amount of amlodipine is about 0.001 mg/kg/day to about 10 mg/kg/day and an especially preferred amount of calcium channel blocker is about 0.01 mg/kg/day to about 5 mg/kg/day.

This invention makes a significant contribution to the field of anti-arrhythmic agents by a new treatment regimen: pretreatment of atrial fibrillation patients with an appropriate calcium channel blocker prior to cardioversion (pharmacological or electrical) improves maintenance of sinus rhythm in conjunction with administration of dofetilide by lowering the likelihood of early recurrence of atrial fibrillation post cardioversion. This treatment regimen has the additional effect of increasing dofetilide's pharmacological conversion rates.

The term arrhythmia refers to conditions in which the normal rhythm of the heart, particularly the sinus rhythm varies.

The term "reduction" is intended to include partial prevention or prevention which, although greater than that which would result from taking no drug or from taking placebo, is less than 100% in addition to substantially total prevention.

The term "treating", "treat" or "treatment" as used herein includes preventative (e.g., prophylactic) and palliative treatment.

The expression "pharmaceutically-acceptable salt" refers to nontoxic anionic salts containing anions such as (but not limited to) chloride, bromide, iodide, sulfate, bisulfate, phosphate, acetate, maleate, fumarate, oxalate, lactate, tartrate, citrate, besylate, gluconate, methanesulfonate and 4-toluene-sulfonate. The expression also refers to nontoxic cationic salts such as (but not limited to) sodium, potassium, calcium, magnesium, ammonium or protonated benzathine (N,N'dibenzylethylenediamine), choline, ethanolamine, diethanolamine, ethylenediamine, meglamine (N-methyl-glucamine), benethamine (N-benzylphenethylamine), piperazine or tromethamine (2-amino-2-hydroxymethyl-1,3-propanediol).

As used herein, the expressions "reaction-inert solvent" and "inert solvent" refers to a solvent or mixture of solvents which does not interact with starting materials, reagents, intermediates or products in a manner which adversely affects the yield of the desired product.

The chemist of ordinary skill will recognize that certain compounds of this invention will contain one or more atoms which may be in a particular stereochemical or geometric configuration, giving rise to stereoisomers and configurational isomers. All such isomers and mixtures thereof are included in this invention. Hydrates and solvents of the compounds of this invention are also included.

Other features and advantages will be apparent from the specification and claims which describe the invention.

### DETAILED DESCRIPTION OF THE INVENTION

As mentioned above dofetilide is known in the art. Dofetilide is claimed and its preparation is described in U.S. Pat. No. 4,959,366, the disclosure of which is hereby incorporated by reference. The following description is provided as an aid to the preparation of dofetilide. Dofetilide has Formula I below and may be named as methanesulfonamide, N-[4-[2-[methyl[2-[4-(methylsulfonyl)amino]phenoxy] ethyl]amino]phenyl]- or β[(p-Methanesulfonamidophenethyl)methylamino]methane-sulfono-p-phenetidide.

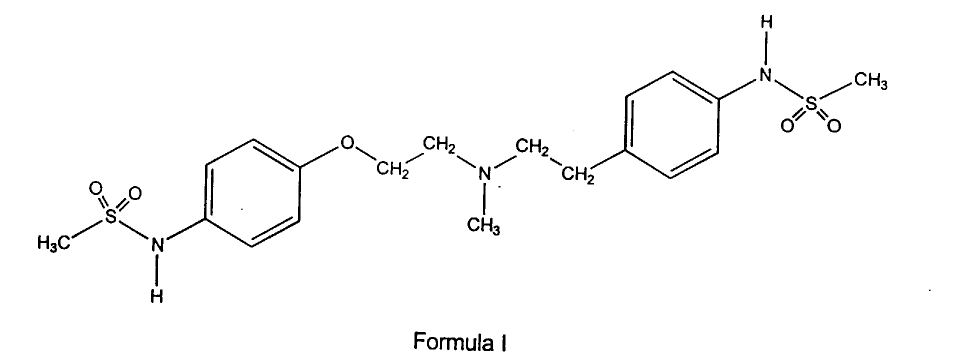

According to U.S. Pat. No. 4,959,366 a solution of 1-(4-amino-phenoxy)-2-[N-(4-aminophenethyl)-N-methylamino]ethane (0.75 g) and methanesulfonic anhydride (1.0 g) in dry methylene chloride (50 ml) was stirred at room temperature overnight. After evaporation, the resultant oil was partitioned between a 2N aqueous sodium bicarbonate solution and ethyl acetate. After two further extractions with ethyl acetate, the organic portions were combined, dried over magnesium sulphate, filtered and evaporated. The resultant colorless solid (1.2 g) was crystallized from ethyl acetate/methanol to give dofetilide. 1-(4-aminophenoxy)-2-[N-(4-aminophenethyl)-N-methylamino]ethane was prepared from a solution of 1-(4-nitrophenoxy)-2-[N-methyl-N-(4-nitrophenethyl)amino]ethane (1.5 g) in ethanol (100ml). The solution was stirred at room temperature under three atmospheres of hydrogen in the presence of Raney nickel. The reaction mixture was filtered and evaporated to dryness. The residual oil was re-dissolved in ether, filtered and evaporated to give a yellow solid (1.1 g), which was crystallized from ethyl acetate/ 60°C-80°C petroleum ether to give the desired product, (0.9g), m.p. 73-74°C.

The starting materials and reagents for the above described synthesis, are also readily available or can be easily synthesized by those skilled in the art using conventional methods of organic synthesis.

Dofetilide is basic and it forms salts with pharmaceutically acceptable anions. All such salts are within the scope of this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

Any calcium channel blocker may be used as the second compound (active agent) of this invention. By "calcium channel blocker" is meant any compound which blocks the entry of Ca²⁺ into cells. Alternatively stated, the term calcium channel blocker refers to compounds which block the movement of Ca²⁺ through the slow, or Ca²⁺, channel and thereby alters the plateau phase of the cardiac action potential. Calcium channels are membrane-spanning, multisubunit proteins that allow controlled entry of Ca²⁺ ions into cells from the extracellular fluid. Briefly, depolarization in atrial tissue is mediated by two inwardly directed ionic currents. When the transmembrane potential of a cardiac cell reaches threshold, there is a rapid influx of Na⁺. The second inward current is caused in large part by the movement of Ca²⁺ into the cell through the slow channel, or Ca²⁺ channel. The influx of Ca²⁺ contributes to the maintenance of the plateau phase of the cardiac action potential.

The most common type of calcium channel is voltage-dependent. In a voltage-dependent channel, the "opening" which allows there to begin an influx of Ca²⁺ ions into the cells requires a depolarization to a certain level of the potential difference between the inside of the cell bearing the channel and the extracellular medium bathing the cell. The rate of influx of Ca²⁺ into the cell depends on this potential difference. Many of these calcium channel blockers bind to calcium channels and block, or reduce the rate of, influx of Ca²⁺ into cells in response to depolarization of the inside and outside of the cells.

Such inhibition is readily determined by those skilled in the art according to standard assays.

In one assay the ability of such compounds to inhibit the movement of calcium into the cell is shown by their effectiveness in reducing the response of isolated heart tissue to an increase in calcium ion concentration in vitro. The test is performed by mounting spirally cut strips of rat aorta with one end fixed and the other attached to a force transducer. The tissue is immersed in a bath of physiological saline solution containing potassium ions at a concentration of 45 millimolar and no calcium. Calcium chloride is added to the bath with a pipette to give a final calcium ion concentration of two millimolar. The change in tension caused by the resulting contraction of the tissue is noted. The bath is drained and replaced with fresh saline solution and, after 45 minutes the test is repeated with the particular compound under test present in the saline solution. The concentration of compound required to reduce the response by 50% is recorded.

The calcium channel blocker can be of any type, and is most preferably a dihydropyridine. Examples of dihydropyridines include amlodipine, nifedipine, nitrendipine, nicardipine, nimodipine, niludipine, riodipine, felodipine, darodipine, isradipine, lercanidipine, and nisoldipine and the pharmaceutically acceptable salts thereof.

Other calcium channel blockers include phenylalkylamines (e.g., verapamil, desmethoxyverapamil, methoxyverapamil), benzothiazepines (e.g., diltiazem), diphenylpiperazines, diarylaminopropylamines (bepridil) and gallopamil and the pharmaceutically acceptable salts thereof.

Amlodipine or a pharmaceutically acceptable salt (e.g., besylate salt) thereof is preferred. Amlodipine is claimed and disclosed in U.S. Pat. No. 4,572,909 and the besylate salt thereof is claimed and disclosed in U.S. Pat. No. 4,879,303, the disclosures of which are hereby incorporated by reference. Amlodipine besylate is also known as 3,5-pyridinedicarboxylic acid, 2-[(2-aminoethoxy)methyl]A-(2-chlorophenyl)1,4-dihyddro-6-methyl-,3-ethyl 5-methyl ester, (±)-, monobenzenesulfonate.

The calcium channel blocker compounds of this invention are readily available or can be easily synthesized by those skilled in the art using conventional methods of organic synthesis.

Some of the compounds of this invention have asymmetric carbon atoms and therefore are enantiomers or diastereomers. Diasteromeric mixtures can be separated into their individual diastereomers on the basis of their physical chemical differences by methods known per se., for example, by chromatography and/or fractional crystallization. Enantiomers can be separated by converting the enantiomeric mixture into a diasteromeric mixture by reaction with an appropriate optically active compound (e.g., alcohol), separating the diastereomers and converting (e.g., hydrolyzing) the individual diastereomers to the corresponding pure enantiomers. All such isomers, including diastereomers, enantiomers and mixtures thereof are considered as part of this invention.

Many calcium channel blockers are acidic or basic and they form a salt with a pharmaceutically acceptable cation or anion as appropriate. All such salts are within the scope of this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

In addition, when the compounds of this invention form hydrates or solvates they are also within the scope of the invention.

The utility of the compounds and combinations of the present invention in the treatment of arrhythmias such as atrial fibrillation in mammals (e.g. humans) is demonstrated by the activity of the compounds and combinations of this invention in conventional assays and the clinical protocol described below. Such assays and clinical protocol also provide a means whereby the activities of the compound(s) of this invention can be compared with the activities of other known compounds. The results of these comparisons are useful for determining dosage levels in mammals, including humans, for the treatment of such diseases.

### PROTOCOL

The recurrence rate of atrial fibrillation was analyzed in a group of 264 patients from a dofetilide phase III study who had been cardioverted from sustained atrial fibrillation of 2-26 weeks duration.

Study #115-120 was a randomized, double-blind, placebo-controlled, parallel group study conducted at multiple centers. Hospitalized patients were given dofetilide bid (125 mcg, 250 mcg, 500 mcg) or placebo according to a 1:1:1:1 randomization. Patients who had not pharmacologically converted after 5 doses were electrically converted to sinus rhythm. Those who remained in sinus rhythm for 24 hours after conversion by either means were considered "converters" and entered a 12 month evaluation of maintenance of sinus rhythm. Patients who relapsed to atrial fibrillation (relapse to atrial fibrillation or flutter lasting at least 24 hours) were considered to have had an endpoint event. The Kaplan-Meier method was used to estimate the probability of sinus rhythm maintenance over time and displayed graphically. Logrank tests were used to test for differences in the sinus rhythm maintenance between the groups and hazard ratios provided from the Cox proportional hazards model. Patients who either completed the 12 months without relapse or discontinued for reasons other than atrial fibrillation relapse were censored in the time-to-event analysis at the day of their last dose of study medication.

The effect of preconversion calcium channel blocker treatment on maintenance of sinus rhythm was analyzed. The 264 patients who converted were analyzed according to their calcium channel blocker use rather than randomized treatment group. The calcium channel blocker group consisted of patients (n=48) who received a calcium channel blocker while in a trial fibrillation from at least 7 days before conversion to at least the day before conversion. The non-calcium channel blocker group consisted of all other converters (n=216). Both groups consisted of patients from all four randomized treatment groups (3 doses of dofetilide and placebo). The groups were compared by the Kaplan-Meier method, logrank test stratified by treatment group and the Cox proportional hazards model with treatment and calcium channel blocker use.

In the patients receiving calcium channel blocker and randomized dofetilide therapy while in atrial fibrillation up to conversion the hazard ratio (relative risk) for atrial fibrillation recurrence through one year adjusted for antiarrhythmic use (randomized treatment group) compared to those not receiving calcium channel blocker and only their randomized dofetilide therapy was 0.77 (95% Cl 0.51 - 1.2). This risk reduction was greatest in the first 15 days after cardioversion, as can be seen from the following Kaplan-Meier estimates of the atrial fibrillation recurrence rates:

| Recurrence rate (%): | | | |
|---|---|---|---|
| [w/ standard error] | 15 days | 30 days | 60 days |
| CCB | (n=48) | 27.4 [0.057] | 38.1 [0.071] 42.6 [0.072] |
| No CCB | (n=216) | 43.0 [0.035] | 48.0 [0.035] 52.1 [0.035] |

Thus, the data demonstrates an improved risk reduction for patients receiving calcium channel blocker treatment and randomized dofetilide treatment vs. those patients receiving only randomized dofetilide treatment.

Furthermore, risk reduction is stronger for the calcium channel blockers amlodipine, nifedipine, nicardipine, or isradipine (and dofetilide) compared to the calcium channel blockers verapamil or diltiazem (and dofetilide) [RR=0.58, (0.29-1.14) p=0.11].

Accordingly, calcium channel blockers have a class dependent protective effect against atrial fibrillation-induced electrical remodeling. Consequently, pretreatment of atrial fibrillation patients with an appropriate calcium channel blocker prior to cardioversion (pharmacological or electrical) improves maintenance of sinus rhythm in conjunction with administration of dofetilide by lowering the likelihood of early recurrence of a trial fibrillation post cardioversion. This treatment regimen has the additional effect of increasing dofetilide's pharmacological conversion rates and decreasing the recurrence rate.

Administration of the compounds and combinations of this invention can be via any method which delivers such compounds and combinations, to the desired tissue (e.g., cardiac tissues). These methods include oral routes, parenteral, intraduodenal routes, etc. Generally, the compounds and combinations of the present invention are administered in single (e.g., once daily) or multiple doses.

The compounds, combinations and methods of this invention are useful in treating arrhythmia such as atrial fibrillation and maintaining normal sinus rhythm. Thus, the compounds, combinations and methods of this invention are useful for improving sinus rhythm in patients at risk for a recurrence of atrial fibrillation post cardioversion. In addition, the compounds, combinations and methods decrease the risk of recurrence of intermittent or paradoxical atrial fibrillation.

Generally, the compounds and combinations of this invention are administered orally, but parenteral administration (e.g., intravenous, intramuscular, subcutaneous or intramedullary) may be utilized, for example, where oral administration is inappropriate for the instant target or where the patient is unable to ingest the drug. Topical administration may also be indicated, for example, where the patient is suffering from gastrointestinal disorders or whenever the medication is best applied to the surface of a tissue or organ as determined by the attending physician.

Typically, the calcium channel blocker is administered in a standard administration mode (e.g., amlodipine is administered once daily) and dofetilide is administered twice daily. Preferably, the patient is pre-treated with the calcium channel blocker for a period of about one day to about one year, with three days to about two weeks being especially preferred, before being converted to normal sinus rhythm. Conversion to normal sinus rhythm is accomplished with administration of dofetilide (with optional electrocardioversion if necessary) and co-administration of the calcium channel blocker for about one to fourteen days followed by long term dofetilide treatment. Optionally, the calcium channel blocker may be continued long term. Of course, when the two compounds are to be administered simultaneously they may be administered together or at different times during the day. Thus, the two different compounds of this invention can be co-administered simultaneously or sequentially in any order, or a single pharmaceutical composition comprising dofetilide and a calcium channel blocker in a pharmaceutically acceptable carrier can be administered.

In any event the amount and timing of compounds administered will, of course, be dependent on the subject being treated, on the severity of the affliction, on the manner of administration and on the judgement of the prescribing physician. Thus, because of patient to patient variability, the dosages given below are a guideline and the physician may titrate doses of the compounds to achieve the treatment (e.g., antiarrhythmic effect) that the physician considers appropriate for the patient.

In general an amount of a compound or combination of this invention is used that is sufficient to attain the desired maintenance of sinus rhythm.

In general an effective dosage for the activities of this invention, for example the antiarrhythmic activities of dofetilide is in the range of 0.1 mcg/kg/day to about 30 mcg/kg/day and an especially preferred amount of dofetilide is about 1.0 mcg/kg/day to about 15 mcg/kg/day, with 15 mcg/kg/day being a preferred dosage.

An amount of the calcium channel blocker of this invention that is effective for the activities of this invention, for example the antiarrhythmic activities is used. Typically, an effective dosage for the calcium channel blockers of this invention is in the range of about 0.001 mg/kg/day to about 10 mg/kg/day in single or divided doses, preferably about 0.1 mg/kg/day to about 5 mg/kg/day in single or divided doses.

The compounds and combinations of the present invention are generally administered in the form of a pharmaceutical composition comprising at least one of the compounds of this invention together with a pharmaceutically acceptable vehicle or diluent. Thus, the compounds and combinations of this invention can be administered individually or together in any conventional oral, parenteral, rectal or transdermal dosage form.

For oral administration a pharmaceutical composition can take the form of solutions, suspensions, tablets, pills, capsules, powders, and the like. Tablets containing various excipients such as sodium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the compounds of this invention can be combined with various sweetening agents, flavoring agents, coloring agents, emulsifying agents and/or suspending agents, as well as such diluents as water, ethanol, propylene glycol, glycerin and various like combinations thereof.

For purposes of parenteral administration, solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well-known to those skilled in the art.

For purposes of transdermal (e.g., topical) administration, dilute sterile, aqueous or partially aqueous solutions (usually in about 0.1% to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples of methods of preparing pharmaceutical compositions, see Remington's Pharmaceutical Sciences, Mack Publishing Company, Easter, Pa., 15th Edition (1975).

Pharmaceutical compositions according to this invention may contain 0.1%-95% of the compound(s) of this invention, preferably 1%-70%. In any event, the composition or formulation to be administered will contain a quantity of a compound(s) according to the invention in an amount effective to treat the disease/ condition of the subject being treated.

Since the present invention has an aspect that relates to the treatment of for example, arrhythmias by treatment with a combination of active ingredients which may be administered separately, the invention also relates to combining separate pharmaceutical compositions in kit form. The kit comprises two separate pharmaceutical compositions: dofetilide and a calcium channel blocker as described above. The kit comprises a means for containing the separate compositions such as a divided bottle or a divided foil packet. Typically the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen which the tablets or capsules so specified should be ingested. Another example of such a memory aid is a calendar printed on the card e.g., as follows "First Week, Monday, Tuesday, ...etc.... Second Week, Monday, Tuesday,..." etc. Other variations of memory aids will be readily apparent. A "daily dose" can be a single tablet or capsule or several tablets or capsules to be taken on a given day. Also a daily dose of the first compound can consist of one tablet or capsule while a daily dose of the second compound can consist of several tablets or capsules and vice versa. The memory aid should reflect this.

In another specific embodiment of this invention a dispenser designed to dispense the daily doses one at a time in the order of their intended use is provided. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

The compounds of this invention either alone or in combination with each other or other compounds generally will be administered in a convenient formulation. The following formulation examples are illustrative only and are not intended to limit the scope of the present invention.

In the formulations which follow, "active ingredient" means compound(s) of this invention and thus may refer to dofetilide, a calcium channel blocker or a combination of the two.

### Formulation 1: Gelatin Capsules

Hard gelatin capsules are prepared using the following:

| Ingredient | Quantity (mg/capsule) |
|---|---|
| Active ingredient | 0.25-100 |
| Starch, NF | 0-650 |
| Starch flowable powder | 0-50 |
| Silicone fluid 350 centistokes | 0.15 |

A tablet formulation is prepared using the ingredients below:

### Formulation 2: Tablets

| Ingredient | Quantity (mg/tablet) |
|---|---|
| Active ingredient | 0.25-100 |
| Cellulose, microcrystalline | 200-650 |
| Silicon dioxide, fumed | 10-650 |
| Stearate acid | 5.15 |

The components are blended and compressed to form tablets.

Alternatively, tablets each containing 0.25-100 mg of active ingredients are prepared as follows:

### Formulation 3: Tablets

| Ingredient | Quantity (mg/tablet) |
|---|---|
| Active ingredient | 0.25-100 |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

The active ingredients, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50° - 60°C and passed through a No. 18 mesh U.S. sieve. The sodium carboxymethyl starch, magnesium stearate, and talc, previously passed through a No. 60 U.S. sieve, are then added to the granules which, after mixing, are compressed on a tablet machine to yield tablets.

Suspensions each containing 0.25-100 mg of active ingredient per 5 ml dose are made as follows:

### Formulation 4: Suspensions

| Ingredient | Quantity (mg/5 ml) |
|---|---|
| Active ingredient | 0.25-100 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified Water to | 5 mL |

The active ingredient is passed through a No. 45 mesh U.S. sieve and mixed with the sodium carboxymethyl cellulose and syrup to form smooth paste. The benzoic acid solution, flavor, and color are diluted with some of the water and added, with stirring. Sufficient water is then added to produce the required volume.

An aerosol solution is prepared containing the following ingredients: Formulation 5: Aerosol

| Ingredient | Quantity (% by weight) |
|---|---|
| Active ingredient | 0.25 |
| Ethanol | 25.75 |
| Propellant 22 (chlorodifluoromethane) | 70.00 |

The active ingredient is mixed with ethanol and the mixture added to a portion of propellant 22, cooled to 30°C, and transferred to a filling device. The required amount is then fed to a stainless steel container and diluted with the remaining propellant. A valve unit is then fitted to the container.

Suppositories are prepared as follows:

### Formulation 6: Suppositories

| Ingredient | Quantity (mg/suppository) |
|---|---|
| Active ingredient | 250 |
| Saturated fatty acid glycerides | 2,000 |

The active ingredient is passed through a No. 60 mesh U.S. sieve and suspended in the saturated fatty acid glycerides previously melted using the minimal necessary heat. The mixture is then poured into a suppository mold of nominal 2 g capacity and allowed to cool.

An intravenous formulation is prepared as follows:

Suppositories are prepared as follows:

### Formulation 7: Intravenous Solution

| Ingredient | Quantity |
|---|---|
| Active ingredient | 20 mg |
| Isotonic saline | 1,000 mL |

The above solution intravenously administered to a patient at a rate of about 1 mL per minute.

## Claims

1. A pharmaceutical composition comprising a therapeutically effective amount of:
a. dofetilide;
b. a calcium channel blocker; and
c. a pharmaceutical acceptable diluent or carrier.

2. A pharmaceutical composition as recited in claim 1, wherein the calcium channel blocker is amlodipine or a pharmaceutically acceptable salt thereof.

3. A pharmaceutical composition as recited in claim 3, wherein the amount of dofetilide is about 0.1 mcg/kg/day to about 30 mcg/kg/day.

4. A pharmaceutical composition as recited in claim 4, wherein the calcium channel blocker is amlodipine besylate and the amount of amlodipine besylate is about 0.01 mg/kg/day to about 5 mg/kg/day.

5. Use of dofetilide for preparation of a medicament comprising or for use in conjunction with a calcium channel blocker for treating a mammal having arrhythmias.

6. Use of a calcium channel blocker for preparation of a medicament for use in conjunction with dofetilide for treatment of arrhythmias.

7. A use as recited in claim 5 or 6, wherein the calcium channel blocker amlodipine or a pharmaceutically acceptable salt thereof.

8. A use as recited in claim 7, wherein the amount of dofetilide is appropriate for a dose of about 0.1 mcg/kg/day to about 30 mcg/kg/day.

9. A use as recited in claim 8, wherein the calcium channel blocker is amlodipine besylate and the amount of amlodipine besylate is appropriate for a dose of about 0.01 mg/kg/day to about 5 mg/kg/day.

10. A use as recited in claim 9, wherein the medicament is for use on a human that has atrial fibrillation and normal sinus rhythm is maintained.

11. A use as recited in claim 10, wherein the medicament is for use on a human whose electrical remodeling of the atrium is inhibited prior to conversion to sinus rhythm.

12. A use as recited in claim 10, wherein the medicament is for a human pretreated with amlodipine or a pharmaceutically acceptable salt thereof prior to conversion to normal sinus rhythm with dofetilide and optional electrocardioversion to result in inhibition of a recurrence of atrial fibrillation post cardioversion.

13. A kit comprising:
a. a therapeutically effective amount of dofetilide and a pharmaceutically acceptable carrier in a first unit dosage form;
b. a therapeutically effective amount of a calcium channel blocker and a pharmaceutically acceptable carrier in a second unit dosage form; and
c. means for containing said first and second dosage forms.

14. A kit as recited in claim 13, wherein the calcium channel blocker is amlodipine or a pharmaceutically acceptable salt thereof.

15. A kit as recited in claim 14, wherein the amount of dofetilide is about 0.1 mcg/kg/day to about 30 mcg/kg/day.

16. A kit as recited in claim 15, wherein the calcium channel blocker is amlodipine besylate and the amount of amlodipine besylate is about 0.01 mg/kg/day to about 5 mg/kg/day.

17. A pharmaceutical composition for treating arrhythmias in a mammal comprising
a. an amount of dofetilide; and
b. an amount of a calcium channel blocker
wherein the amount of dofetilide alone and the amount of the calcium channel blocker alone is insufficient to achieve the improved maintenance of normal sinus rhythm if administered alone and wherein the combined effect of the amount of dofetilide and the calcium channel blocker is greater than the sum of the sinus rhythm maintenance effects achievable with individual amounts of dofetilide and calcium channel blocker; and
c. a pharmaceutically acceptable diluent or carrier.

18. A pharmaceutical composition as recited in claim 17. wherein the calcium channel blocker is amlodipine or a pharmaceutically acceptable salt thereof.

19. A pharmaceutical composition as recited in claim 18, wherein the amount of dofetilide is about 0.1 mcg/kg/day to about 30 mcg/kg/day.

20. A pharmaceutical composition as recited in claim 19, wherein the calcium channel blocker is amlodipine besylate and the amount of amlodipine besylate is about 0.01 mg/kg/day to about 5 mg/kg/day.

21. Use of dofetilide for preparation of a medicament comprising or for use in conjunction with a calcium channel blocker for improving the maintenance of sinus rhythm in a mammal wherein the amount of dofetilide alone and the amount of the calcium channel blocker alone is insufficient to achieve the improved maintenance of normal sinus rhythm if administered alone and wherein the combined effect of the amount of dofetilide and the calcium channel blocker is greater than the sum of the sinus rhythm maintenance effects achievable with individual amounts of the dofetilide and calcium channel blocker.

22. A use as recited in claim 20, wherein the calcium channel blocker is amlodipine or a pharmaceutically acceptable salt thereof.

23. A use as recited in claim 22, wherein the amount of dofetilide is appropriate for a dose of about 0.1 mcg/kg/day to about 30 mcg/kg/day.

24. A use as recited in claim 22, wherein the calcium channel blocker is amlodipine besylate and the amount of amlodipine besylate is appropriate for a dose of about 0.01 mg/kg/day to about 5 mg/kg/day.

25. A use as recited in claim 24, wherein the medicament is for use in a human.

26. A use of amlodipine for production of a medicamemt for treating arrhythmias in a mammal or a pharmaceutically acceptable salt thereof.

27. A use as recited in claim 26, wherein the medicament is one whereby atrial fibrillation is treated.

28. A use as recited in claim 26, wherein the medicament is one whereby maintenance of normal sinus rhythm is improved.

29. A use as recited in claim 26, wherein the medicament is one whereby electrical remodeling of the atrium prior to conversion to sinus rhythm is inhibited.

30. A use as recited in claim 26, wherein the medicament is for a human.

31. A use as recited in claim 30, wherein the calcium channel blocker is amlodipine besylate and the amount of amlodipine besylate appropriate for a dose of about 0.01 mg/kg/day to about 5 mg/kg/day.

32. A use as recited in claim 30, wherein the medicament is for a human pretreated with amlodipine or a pharmaceutically acceptable salt thereof prior to conversion to normal sinus rhythm resulting in inhibition of a recurrence of atrial fibrillation post cardioversion.
